# EUROPEAN PATENT APPLICATION

(11) **EP 4 397 410 A1**
(43) Date of publication of application: **10.07.2024**
(21) Application number: 22864917.4
(22) Date of filing: 17.08.2022
(51) Int. Cl.: B01L 3/00, A61B 10/00

(54) **SAMPLE COLLECTION DEVICE AND SAMPLE COLLECTION METHOD**

(30) Priority: 31.08.2021 KR 20210115297
(71) Applicant: Seegene, Inc., Seoul 05548 (KR)
(72) Inventor: KIM, Won Sik, Seongnam-si Gyeonggi-do 13643 (KR); ROH, Kyoung Min, Seoul 05354 (KR); SEO, Myung Jun, Seoul 02071 (KR); UM, Young Il, Seoul 03084 (KR)
(74) Representative: Viering, Jentschura & Partner mbB Patent- und Rechtsanwälte
(86) International application number: PCT/KR2022/012273
(87) International publication number: WO 2023/033413

(57) **Abstract**

The present invention relates to a sample collection device, comprising: (a) a first tube accommodating a sample; wherein the first tube comprises an opened end part; (b) a second tube accommodating a sample transport medium; wherein the second tube comprises an opened end part; and (c) an adapter; wherein the adapter comprises a first accommodating part formed such that the opened end part of the first tube is inserted, a second accommodating part formed to be coupled to the opened end part of the second tube and a sealing part that comprises a first surface facing the first accommodating part and a second surface facing the second accommodating part, and partitions the first accommodating part and the second accommodating part, and wherein a breaking line is formed on the sealing part such that the sealing part is broken by a contact pressure.

## Description

### BACKGROUND OF THE INVENTION

### FIELD OF THE INVENTION

The present embodiments relate to a sample collection device and a method for collecting a sample.

### DESCRIPTION OF THE RELATED ART

As the interest in the health of modern people increases and the life expectancy is extended, the importance of nucleic acid-based *in vitro* molecular diagnosis, such as accurate analysis of pathogens and gene analysis of patients, is increasing, and the demand is increasing.

Nucleic acid-based molecular diagnosis is performed by extracting nucleic acid from a sample, and then confirming whether a target nucleic acid is present among the extracted nucleic acid. With the recent global spread of SARS-CoV-2, real-time PCR diagnostic kits have been receiving a lot of attention, various point-of-care (POC) systems have been developed as the demand for molecular diagnosis by quarantine authorities and the public has gradually changed in the direction of enhancing rapidity and convenience due to the prolonged SARS-CoV-2 pandemic, and in particular, new types of molecular diagnostic home test products have been released mainly in the United States, guidelines for such home test have been established, and the general public can simply and safely perform home test in daily life.

In order to perform on-site diagnosis and home test as well as PCR diagnosis at a testing site such as a medical institution, a device for collecting the collected sample is required, and in order to perform molecular diagnosis with improved speed and convenience, not only an expert but also a patient (general person) who wants to receive diagnosis needs a sample collection device capable of collecting the sample by itself.

Accordingly, the present inventors have recognized the needs for the development of a sample collection device that is convenient for use by the general public and can safely protect the general public collecting the sample from pathogens included in the sample or chemical substances included in the sample transport medium.

Therefore, there is a need for development of a sample collection device which can be conveniently used and can protect a human body from a collected sample or a chemical material included in a sample transport medium.

### SUMMARY OF THE INVENTION

To overcome the problems of the prior art, the present invention provides a sample collection device that can be used safely and conveniently.

In addition, the present invention provides a sample collection device capable of easily supplying a sample transport medium to a collected sample and preventing the sample transport buffer from leaking in the process of supplying the sample transport medium to the sample, thereby being safe.

In an aspect of the present invention, there is provided a sample collection device, comprising: (a) a first tube accommodating a sample; wherein the first tube comprises an opened end part; (b) a second tube accommodating a sample transport medium; wherein the second tube comprises an opened end part; and (c) an adapter; wherein the adapter comprises a first accommodating part formed such that the opened end part of the first tube is inserted, a second accommodating part formed to be coupled to the opened end part of the second tube and a sealing part that comprises a first surface facing the first accommodating part and a second surface facing the second accommodating part, and partitions the first accommodating part and the second accommodating part, and wherein a breaking line is formed on the sealing part such that the sealing part is broken by a contact pressure.

In another aspect of the present invention, there is also provided a sample collection device, comprising: (a) a first tube accommodating a sample; wherein the first tube comprises an opened end part; and (b) a second tube accommodating a sample transport medium; wherein the second tube comprises an accommodating part formed at one end of the second tube such that the opened end part of the first tube is inserted and a sealing part that comprises a first surface facing the accommodating part and a second surface facing the other end of the second tube and partitions the second tube, and wherein a breaking line is formed on the sealing part such that the sealing part is broken by a contact pressure.

In still another aspect of the present invention, there is provided a method for collecting a sample, comprising: (a) accommodating a collected sample in a first tube; wherein the first tube comprises an opened end part; (b) inserting the first tube accommodating the sample into an adapter coupled with a second tube accommodating a sample transport medium; wherein the second tube comprises an opened end part, wherein the adapter comprises a first accommodating part formed such that the opened end part of the first tube is inserted, a second accommodating part formed to be coupled to the opened end part of the second tube and a sealing part that comprises a first surface facing the first accommodating part and a second surface facing the second accommodating part, and partitions the first accommodating part and the second accommodating part, and wherein a breaking line is formed on the sealing part such that the sealing part is broken by a contact pressure; and (c) breaking the sealing part and moving the sample transport medium accommodated in the second tube to the first tube.

According to the present invention, it is possible to provide a sample collection device that can be used safely and conveniently.

In addition, according to the present invention, a sample transport medium can be easily supplied to a collected sample, and the sample transport medium is prevented from leaking during a process of supplying the sample transport medium to the sample, thereby providing a safe sample collection device.

In addition, according to the present invention, self-collection of samples is possible easily by an ordinary person without the involvement of an expert.

### BRIEF DESCRIPTION OF THE DRAWINGS

FIG. 1 is a perspective view of a sample collection device according to an embodiment of the present invention.
FIG. 2 is a diagram illustrating an example of use of a sample collection device according to an embodiment of the present invention.
FIG. 3 is a cross-sectional view of a part of a sample collection device according to an embodiment of the present invention.
FIG. 4 is a cross-sectional view of a part of a sample collection device according to an embodiment of the present invention.
FIG. 5 is a bottom view and a cross-sectional view of a part of a sample collection device according to an embodiment of the present invention.
FIG. 6 is a diagram illustrating an operation example of a sample collection device according to an embodiment of the present invention.
FIG. 7 is a perspective view of a part of a sample collection device according to an embodiment of the present invention.
FIG. 8 is a view illustrating an operation example of a sample collection device according to an embodiment of the present invention.
FIG. 9 is a perspective view of a part of a sample collection device according to an embodiment of the present invention.
FIG. 10 is a view illustrating an operation example of a sample collection device according to an embodiment of the present invention.
FIG. 11 is a perspective view of a sample collection device according to an embodiment of the present invention.
FIG. 12 is a perspective view of a sample collection device according to an embodiment of the present invention.
FIG. 13 is a cross-sectional view of a sample collection device according to an embodiment of the present invention.
FIG. 14 is a perspective view of a sample collection device according to an embodiment of the present invention.

### DETAILED DESCRIPTION OF THIS INVETNION

Hereinafter, the present invention will be described in more detail through examples with reference to the drawings. These embodiments are provided only to describe the present invention in more detail, and it will be apparent to those skilled in the art that the scope of the present invention is not limited by these embodiments according to the gist of the present invention.

In addition, in describing the feature element (component) of the present invention, terms such as first, second, A, B, (a), (b), (i), (ii), and the like may be used. The term is used only to distinguish the feature element from other feature elements, and the nature, sequence, or order of the corresponding feature element is not limited by the term. When a feature element is described as being "connected", "coupled" or "joined" to another element, the feature element may be directly connected or joined to the other element, but it should be understood that another feature element may be "connected", "coupled" or " joined" between each feature element.

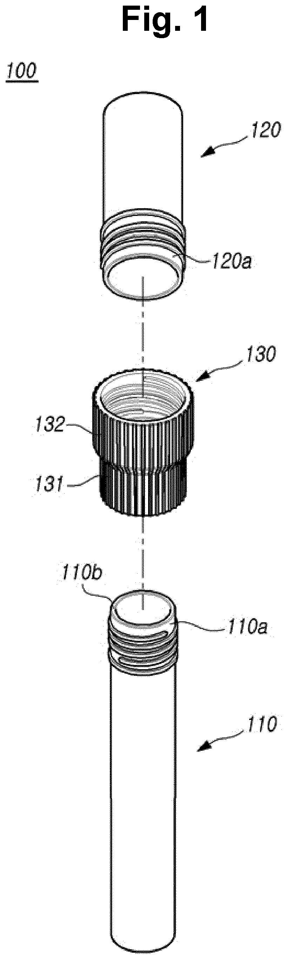

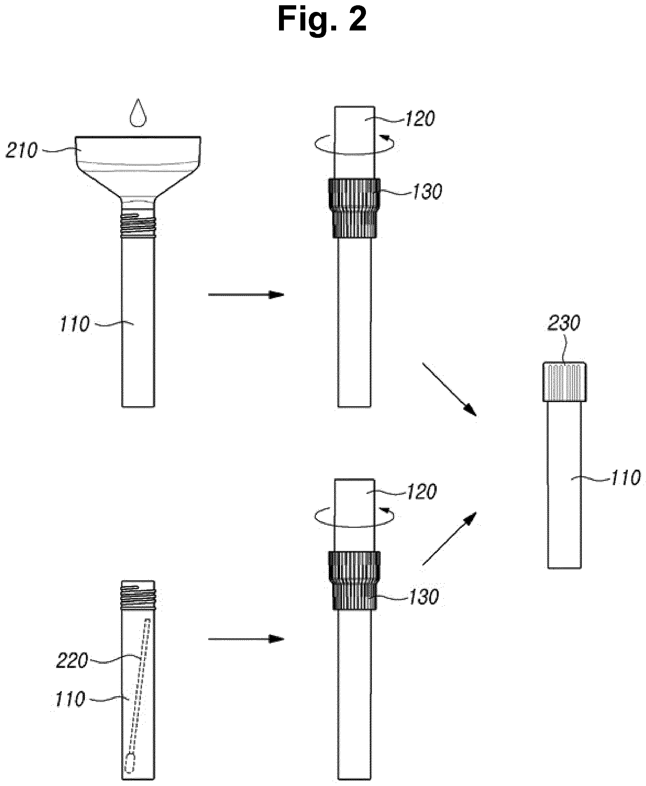

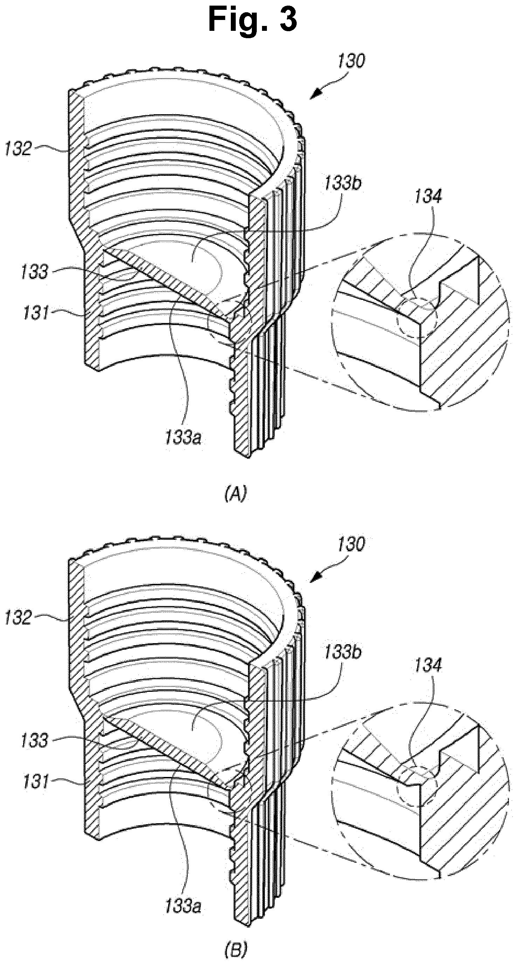

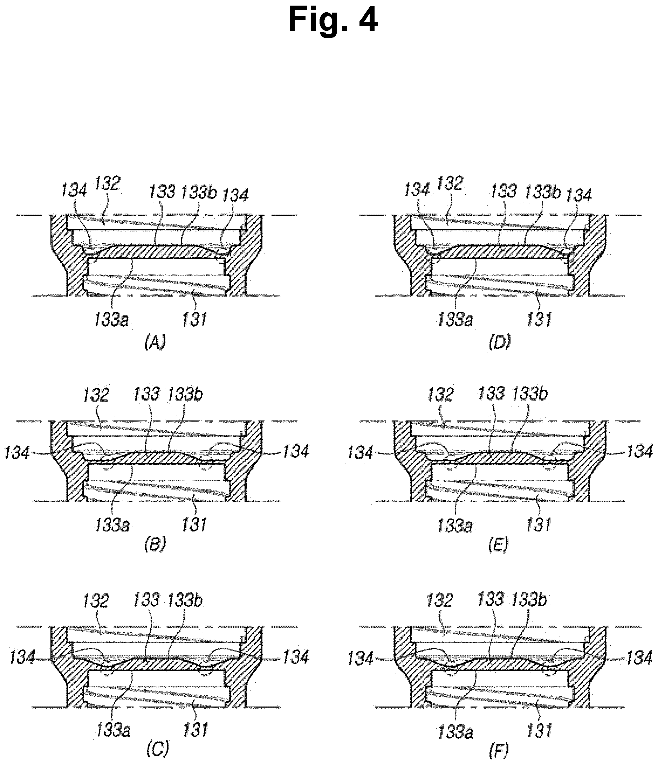

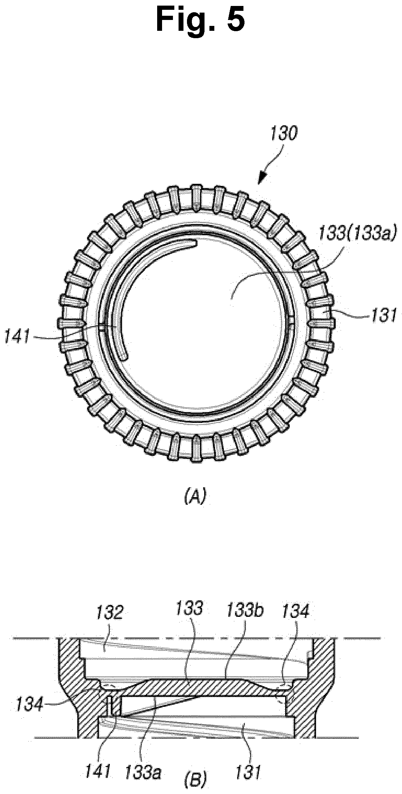

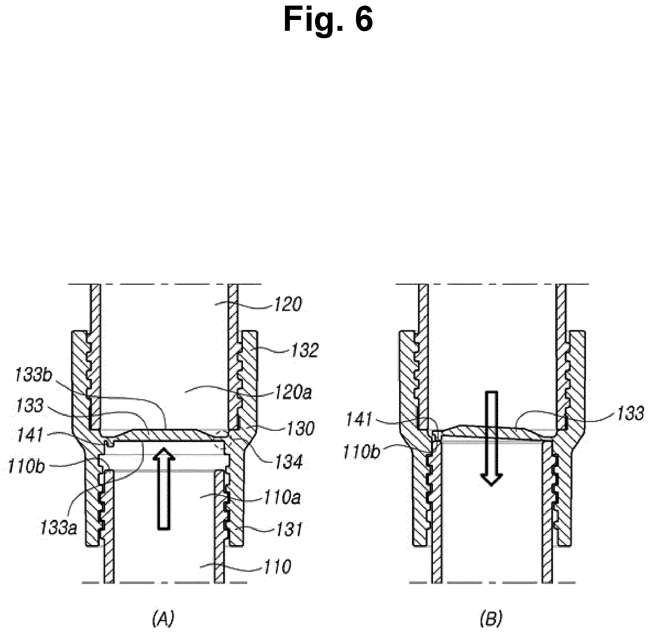

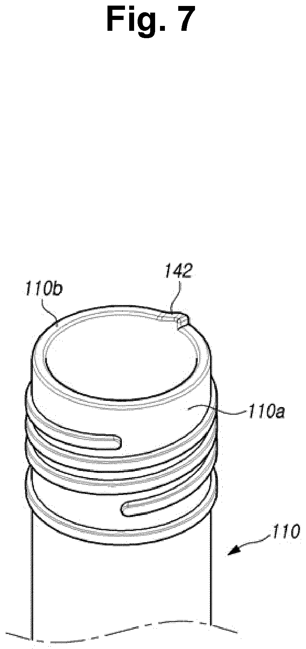

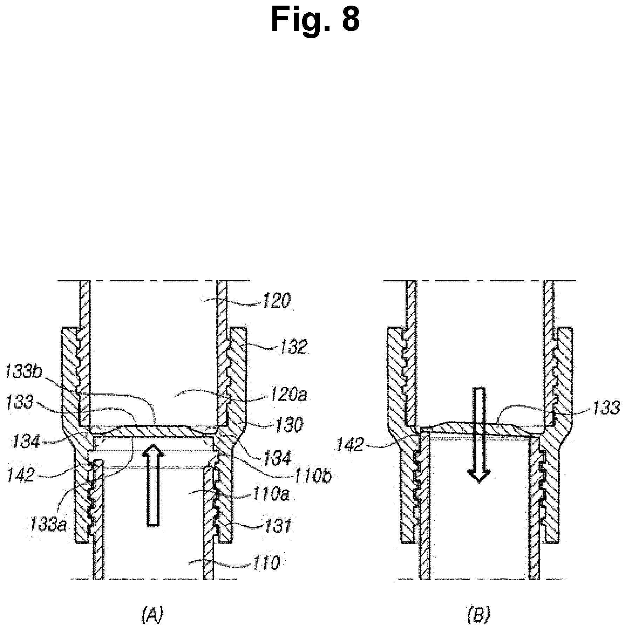

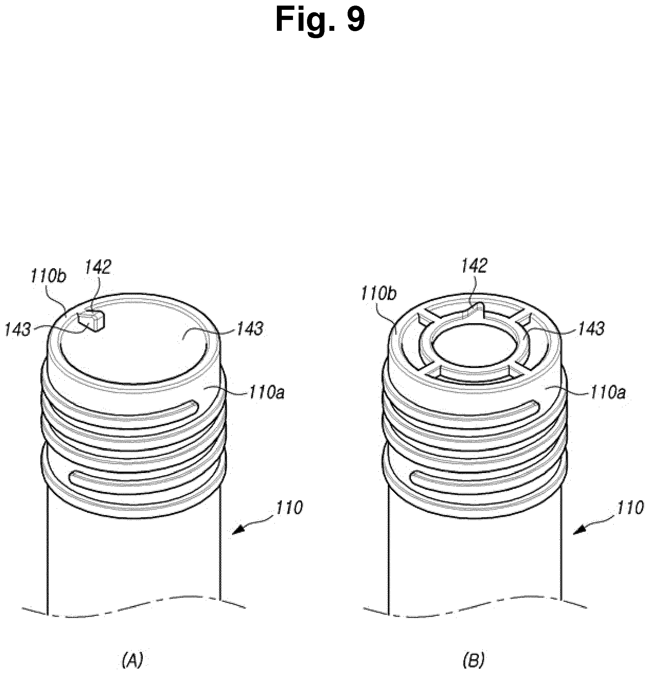

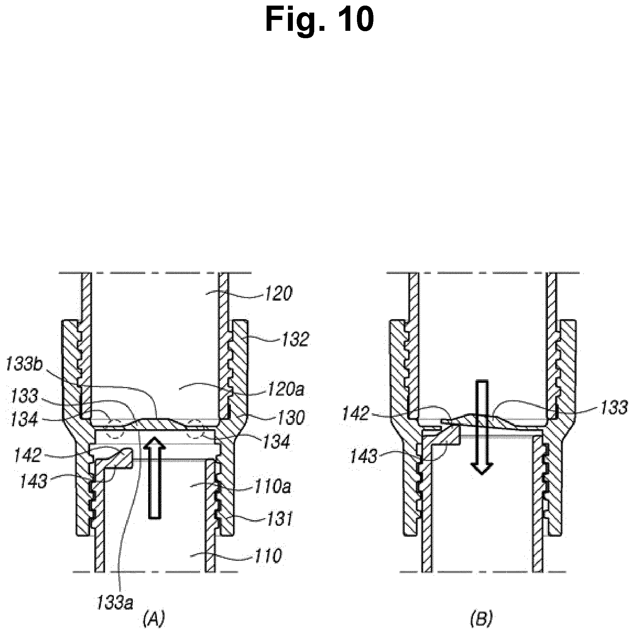

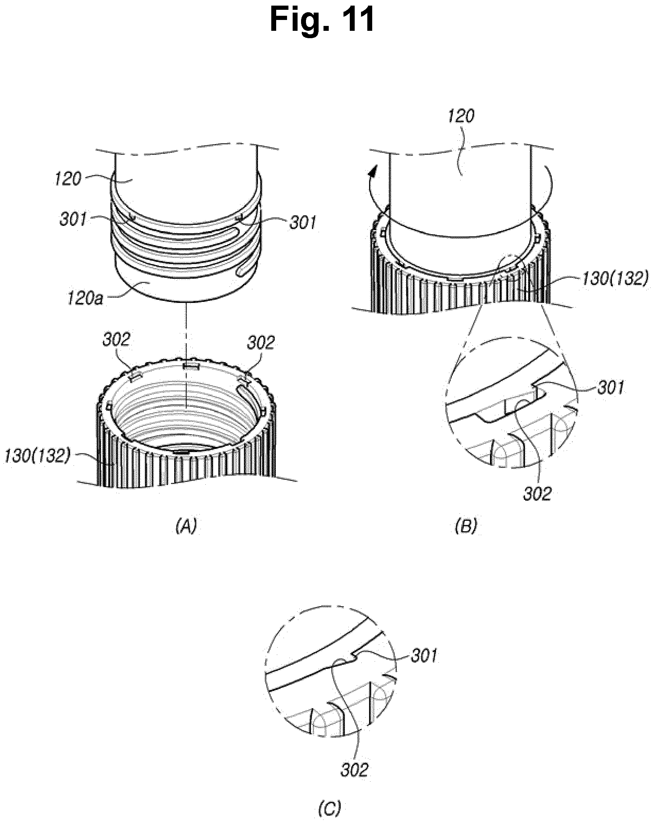

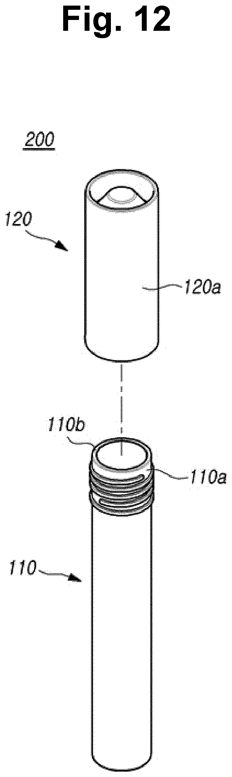

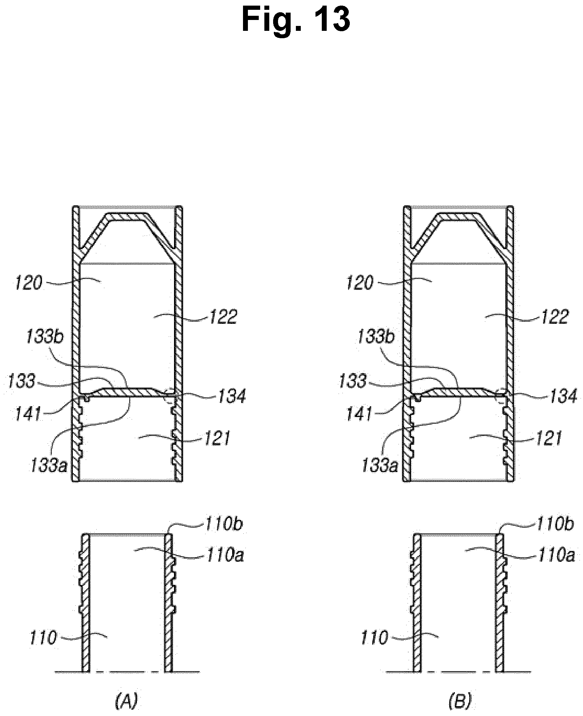

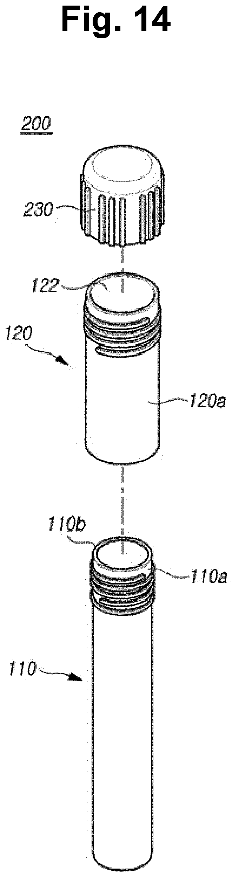

FIG. 1 is a perspective view of a sample collection device according to an embodiment of the present invention, FIG. 2 is a view illustrating an example of use of the sample collection device according to an embodiment of the present invention, FIG. 3 is a cross-sectional view of a part of the sample collection device according to an embodiment of the present invention, FIG. 4 is a cross-sectional view of a part of the sample collection device according to an embodiment of the present invention, FIG. 5 is a bottom view and a cross-sectional view of a part of the sample collection device according to an embodiment of the present invention, FIG. 6 is a view illustrating an operation example of the sample collection device according to an embodiment of the present invention, FIG. 7 is a perspective view of a part of the sample collection device according to an embodiment of the present invention, FIG. 8 is a view illustrating an operation example of the sample collection device according to an embodiment of the present invention, FIG. 9 is a perspective view of a part of the sample collection device according to an embodiment of the present invention, FIG. 10 is a view illustrating an operation example of the sample collection device according to an embodiment of the present invention, FIG. 11 is a perspective view of a sample collection device according to an embodiment of the present invention, FIG. 12 is a perspective view of a sample collection device according to an embodiment of the present invention, FIG. 13 is a cross-sectional view of a sample collection device according to an embodiment of the present invention, FIG. 14 is a perspective view of a sample collection device according to an embodiment of the present invention.

Referring to FIGS. 1 to 11, in an aspect of the present invention, there is provided a sample collection device **100** (the sample collection device of the first aspect), comprising: (a) a first tube **110** accommodating a sample; wherein the first tube **110** comprises an opened end part **110a,** (b) a second tube **120** accommodating a sample transport medium; wherein the second tube **120** comprises an opened end part **120a;** and (c) an adapter **130;** wherein the adapter **130** comprises a first accommodating part **131** formed such that the opened end part **110a** of the first tube **110** is inserted, a second accommodating part **132** formed to be coupled to the opened end part **120a** of the second tube **120,** and a sealing part **133** that comprises a first surface **133a** facing the first accommodating part **131** and a second surface **133b** facing the second accommodating part **132,** and partitions the first accommodating part **131** and the second accommodating part **132,** and wherein a breaking line **134** is formed on the sealing part **133** such that the sealing part **133** is broken by a contact pressure.

Referring to FIG. 1, the sample collection device **100** according to an embodiment of the present invention includes a first tube **110,** a second tube **120,** and an adapter **130.** The first tube **110** is a tube in which a user collects and accommodates (or receives) a sample, the second tube **120** is a tube in which a sample transport medium is accommodated (or included), and the adapter **130** connects the first tube **110** and the second tube **120** and allows the sample transport medium accommodated in the second tube **120** to be supplied to the sample accommodated in the first tube **110.**

The first tube **110** and the second tube **120** respectively include the opened end parts **110a** and **120a,** and the adapter **130** includes the first accommodating part **131** formed such that the opened end part **110a** of the first tube **110** is inserted and a second accommodating part **132** formed to be coupled to the opened end part **120a** of the second tube **120.** As will be described in detail later, according to one embodiment of the present invention, the first tube **110** and the first accommodating part **131** may be screw-coupled. In addition, according to one embodiment of the present invention, the second tube **120** and the second accommodating part **132** may be coupled together by their screw threads.

The sealing part **133** is located inside the adapter **130,** includes the first surface **133a** facing the first accommodating part **131** and the second surface **133b** facing the second accommodating part **132,** and partitions the inside of the adapter **130** into the first accommodating part **131** and the second accommodating part **132.** That is, the adapter **130** has, for example, a hollow shape with both ends opened such that the first tube **110** and the second tube **120** are inserted and coupled and the inside of the adapter **130** is spatially divided into the first accommodating part **131** and the second accommodating part **132** by the sealing part **133.** The breaking line **134** is formed on the sealing part **133** such that the sealing part **133** is broken by a contact pressure, and as the sealing part **133** is broken, the first accommodating part **131** and the second accommodating part **132** are communicated with each other.

Referring to FIG. 2, the user accommodates a sample collected through the opened end part **110a** of the first tube **110** into the first tube **110.** According to an embodiment of the present invention, the sample may be selected from the group consisting of a saliva sample, a saliva swab sample, a nasopharyngeal swab sample, an oropharyngeal swab sample, a nasal sample, a nasal swab sample, a nostril sample, and a nostril swab sample.

In the present specification, "a saliva sample, a nasal sample, or a nostril sample" indicates a sample that is collected without using a swab device for collecting a sample, and "a saliva swab sample, a nasopharyngeal swab sample, an oropharyngeal swab sample, a nasal swab sample, or a nostril swab sample" indicates a sample that is collected using a swab device for collecting a sample.

According to an embodiment, the sample collection device **100** further includes a funnel **210** to be coupled to the opened end part **110a** of the first tube **110,** and the user may accommodate (or receive) a sample, for example, saliva, into the first tube **110** by using the funnel **210.** According to an embodiment of the present invention, the sample collection device **100** may further include a swab device for collecting a sample **220,** and the user may collect a swab sample by using the swab device for collecting a sample **220** and insert the swab device into the first tube **110** to accommodate the swab sample in the first tube **110.**

The sample transport medium accommodated in the second tube **120** is supplied to the second tube **120** through the opened end part **120a** of the second tube **120,** and the second tube **120** is coupled to the adapter **130** to accommodate the sample transport medium. In other words, according to an embodiment of the present invention, the sample transport medium may be accommodated in a space formed by coupling the opened end part **120a** of the second tube **120** and the second accommodating part **132** partitioned by the sealing part **133.** The sample transport medium is a solution for transferring (or transporting) and preserving the collected sample until the test (or inspection) is performed, and the second tube **120** and the adapter **130** may be coupled to each other to be stored and transferred in a state in which the sample transport medium is accommodated and provided to the user.

The sample transport medium includes a maintaining buffer and/or a lysis buffer (*i.e.,* an inactivation buffer). The maintaining buffer refers to a buffer that maintains the sample so as not to be lysed. The lysis buffer is a buffer that lyses *e.g.,* a virus contained in a sample such that nucleic acid molecules in the virus are released into the buffer, denatures protein components and inactivates DNase and RNase, and maintains the integrity of the nucleic acid molecules. More specifically, the sample transport medium is a lysis buffer.

According to a specific example of the present invention, the sample transport medium comprises (i) a chaotropic agent, (ii) a detergent, (iii) a chelator, and (iv) a buffer. Optionally, the sample transport medium may further include a reducing agent.

Specifically, the chaotropic agent is guanidine thiocyanate, guanidine isocyanate, guanidine hydrochloride and potassium thiocyanate, and more specifically, guanidine thiocyanate. The chaotropic agent induces cell lysis by opening microbial cell, causes DNA and RNA to be released, and inhibits nucleic acid molecules from being degradation by nuclease.

Specifically, the detergent is sodium dodecyl sulfate, lithium dodecyl sulfate, sodium taurodeoxycholate, sodium taurocholate, sodium glycocholate, sodium deoxycholate, sodium cholate, sodium alkylbenzene sulfonate, polysorbate, Triton X-100 or N-lauroyl sarcosine.

Specifically, the chelator is ethylene glycol tetraacetic acid, hydroxyethylethylenediaminetriacetic acid, diethylene triamine pentaacetic acid, N,N-bis(carboxymethyl)glycine, ethylenediaminetetraacetic, citrate anhydrous, sodium citrate, calcium citrate, ammonium citrate, ammonium bicitrate, citric acid, diammonium citrate, ferric ammonium citrate or lithium citrate.

Specifically, the buffer is tris(hydroxymethyl)aminomethane, citrate, 2-(N-morpholino)ethanesulfonic acid, N,N-Bis(2-hydroxyethyl)-2-aminoethanesulfonic acid, 1,3-bis(tris(hydroxymethyl)methyl amino)propane, 4-(2-hydroxyethyl)-1-piperazine ethanesulfonic acid, 3-(N-morpholino)propanesulfonic acid, hydroxyethyl piperazine ethane sulfonic acid, bicarbonate and phosphate.

Specifically, the reducing agent is 2-mercaptoethanol, tris(2-carboxyethyl)phosphine, dithiothreitol, dimethylsulfoxide and sodium hydroxide

In the sample transport medium, the chaotropic agent may be included in an amount of 5-30 parts by weight based on (or relative to) 100 parts by weight of the medium, the detergent may be included in an amount of 1-15 parts by weight, the chelator may be included in an amount of 0.1-5 parts by weight, the buffer may be included in an amount of 2-10 parts by weight, and the reducing agent may be included in an amount of 0.1-3 parts by weight.

According to an embodiment of the present invention, the sample transport medium has an inactivation function by lysis of, for example, a respiratory infection pathogen included in a sample and a stabilization function of a nucleic acid material (specifically, DNA or RNA, more specifically RNA) released from the lysed pathogen.

Optionally, the sample transport medium further comprises a pH indicator (*e.g*., phenol red, bromocresol purple and bromothymol blue), and more specifically, phenol red. The pH indicator makes it possible to monitor whether or not the sample transport buffer is infected with bacteria.

Then, the user may accommodate the sample collected in the first tube **110** and then insert the first tube **110** into the adapter **130** to which the second tube **120** is coupled, thereby moving the sample transport medium into the first tube **110.** According to an embodiment of the present invention, the sample transport medium moves from the second tube **120** to the first tube **110** after the sealing part **133** is broken. According to an embodiment, the contact pressure is provided by the first tube **110** inserted into the first accommodating part **131.** Specifically, the contact pressure for breaking or fracturing the sealing part **133** is provided by the first tube **110** inserted into the first accommodating part **131,** and the sample transport medium is moved from the inside of the second tube **120** to the inside of the first tube **110,** and a structure in which the sealing part **133** is broken or fractured will be described in detail later.

According to one embodiment of the present invention, the first tube **110** and the first accommodating part **131** may be coupled together by their screw threads. That is, the user may insert and screw-couple the first tube **110** into the first accommodating part **131.** Specifically, screw threads are formed on an outer circumferential surface of an end part of the first tube **110** and screw threads are formed on an inner circumferential surface of the first accommodating part **131,** so that the first tube **110** may be screw-coupled through the screw threads while being inserted into the first accommodating part **131.**

According to an embodiment, the first tube **110** and/or the adapter **130** have a rib formed on an outer circumferential surface thereof. The user may easily couple or separate the first tube **110** and the adapter **130** by holding the first tube **110** and/or the adapter **130** with both hands and applying a force through the ribs formed on the outer circumferential surface of the first tube **110** and/or the adapter **130.**

According to an embodiment of the present invention, the rib of the first tube **110** is formed on the outer circumferential surface under the threaded end.

According to an embodiment of the present invention, the rib is provided in plurality.

The ribs formed in the first tube **110** and/or the adapter **130** may induce a user collecting a sample to couple the first tube **110** and the adapter **130,** and may also induce an examiner for obtaining a sample for a nucleic acid amplification reaction at a test site to separate the first tube **110** and the adapter **130.**

According to an embodiment, an indicator indicating whether the first tube **110** and the adapter **130** are coupled is formed on outer circumferential surfaces thereof. Specifically, when the first tube **110** and the adapter **130** are coupled, if the indicators formed on the outer circumferential surfaces of the first tube **110** and the adapter **130** coincide with each other, it indicates that they are completely coupled, and if they do not coincide with each other, it indicates that they are not completely coupled. The indicator may be formed on the outer circumferential surfaces of the first tube **110** and the adapter **130** in various shapes of grooves or protrusions, and the shape may be, for example, a circular shape, a straight shape, or a triangular shape.

As the first tube **110** is inserted into the first accommodating part **131,** it is possible to prevent the sample transport medium accommodated in the second tube **120** from being leaked to the outside of the first tube **110** while moving to the first tube **110.**

In this specification, the term "contact pressure" refers to pressure generated by direct or indirect contact between the sealing part **133** and the first tube **110** for breaking the sealing part **133.** The contact pressure in the present invention does not include those cut with a sharp blade.

According to an embodiment of the present invention, the contact pressure for breaking the sealing part **133** may be provided as the first tube **110** is inserted into the first accommodating part **131** and moves forward. That is, when the first tube **110** is inserted into the first accommodating part **131** and moves forward, the first tube **110** contacts the sealing part **133** and a contact pressure is applied thereto, and the first tube **110** continuously moves forward, the contact pressure increases and the sealing part **133** may be broken.

The user may insert the first tube **110** into the adapter **130** to which the second tube **120** is coupled, supply the sample transport medium to the sample, and then transport the first tube **110** accommodating the sample and the sample transport medium to the inspection place. The first tube **110** may be transported to the inspection place in a state of being coupled to the adapter **130** to which the second tube **120** is coupled. Alternatively, as will be described later, the first tube **110** may be separated from the adapter **130** and transported to the inspection site in a state in which a tube cap **230** is coupled to the opened end part **110a** of the first tube **110.**

As used herein, the expression "second accommodating part formed to be coupled to the opened end part of the second tube" means that the opened end part **120a** of the second tube **120** is formed to be inserted into the second accommodating part **132** or the second accommodating part **132** is formed to be inserted into the opened end part **120a** of the second tube **120.**

According to one embodiment, the second tube **120** and the second accommodating part **132** are coupled together by their screw threads. Specifically, screw threads are formed on an outer circumferential surface or an inner circumferential surface of an end of the second tube **120,** and screw threads are formed on an inner circumferential surface or an outer circumferential surface of the second accommodating part **132,** such that the second tube **120** may be screw-coupled by screw threads while being inserted into the second accommodating part **132** or the second accommodating part **132** may be screw-coupled by screw threads while being inserted into the second tube **120.**

In order to obtain a sample for a nucleic acid amplification reaction from a sample collection device transported to a testing site (or an inspection place), the first tube **110** and the adapter **130** should be separated from each other or the first tube **110** and the tube cap **230** should be separated from each other. When the first tube **110** and the adapter **130** are separated from each other, the first tube **110** may be separated from the adapter **130** by respectively holding the first tube **110** and the adapter **130** of the sample collection device with both hands and rotating in a direction opposite to the coupling process of the first tube **110.**

According to one embodiment of the present invention, in order to prevent the adapter **130** and the second tube **120** from being separated from each other after being coupled, a groove **302** and a protrusion **301** may be formed in the adapter **130** and the second tube **120,** respectively. Referring to FIG. 11A, the second tube **120** may include a plurality of protrusions **301** disposed on an outer circumferential surface thereof, and the second accommodating part **132** may include a plurality of grooves **302** disposed on an inner circumferential surface thereof and engaged with the protrusions **301.** The protrusion **301** and the groove **302** are formed to prevent the second tube **120** from being separated from the second accommodating part **132.** That is, when the second tube **120** is inserted into and coupled to the second accommodating part **132,** the protrusion **301** and the groove **302** are engaged with each other to prevent the second tube **120** from being separated from the adapter **130.**

According to an embodiment, the protrusion **301** and the groove **302** are formed to be rotated only in a direction in which the second tube **120** is coupled to the second accommodating part **132.** According to one embodiment of the present invention, when the second tube **120** and the second accommodating part **132** are screw-coupled, the protrusion **301** and the groove **302** may be formed such that the second tube **120** is coupled to the second accommodating part **132** and is rotated only in a forward direction, and is not rotated in a backward direction. That is, the protrusion **301** and the groove **302** may prevent the second tube **120** from being separated from the adapter **130** by being coupled to the second accommodating part **132,** rotating only in one direction and preventing the second tube **120** from rotating in the opposite direction to which the second tube **120** is moving backwards. According to an embodiment, one surface of the protrusion **301** in the circumferential direction is formed in a gentle wedge shape and the other surface thereof is formed in a steep wedge shape, and the groove **302** is formed in a shape into which the protrusion **301** may be inserted at an end of the second accommodating part **132.** As illustrated in (A) and (B) of FIG. 11, the groove **302** may be formed in a shape of an arc-shaped groove, or as illustrated in (C) of FIG. 11, may be formed in a shape engaged with the wedge-shaped protrusion **301.** As the protrusion **301** is inserted into the groove **302,** the second tube **120** may rotate only in one direction and may not rotate in the other direction.

Alternatively, the user may insert the first tube **110** into the adapter **130** coupled with the second tube **120** to supply the sample transport medium to the sample, separate the adapter **130** from the first tube **110,** seal the first tube **110** with the tube cap **230,** and transport the sealed first tube **110** to the testing site.

According to an embodiment of the present invention, the sample collection device **100** may further include a tube cap **230** covering the opened end part **110a** of the first tube **110,** so that the user may seal the first tube **110** by coupling the tube cap **230** to the first tube **110** in which the sample and the sample transport medium are accommodated.

As described above, according to the sample collection device **100** according to the present invention, the user may easily store the sample in the tube together with the sample transport medium through a simple step of accommodating the collected sample in the first tube **110,** inserting the first tube **110** into the adapter **130** coupled to the second tube **120,** and breaking the sealing part **133** to move the sample transport medium. According to the sample collection device **100** of the present invention, only an intuitive operation of coupling and separating the first tube **110** and the adapter **130** of the user is required, and thus the use thereof is very simple.

Hereinafter, a structure in which the sealing part **133** is broken as the first tube **110** is inserted into the adapter **130** and the sample transport medium accommodated in the second tube **120** is moved to the first tube **110** will be described in detail.

Referring to FIGS. 3 and 4, as described above, the adapter **130** includes the first accommodating part **131,** the second accommodating part **132,** and the sealing part **133.** The sealing part **133** includes a first surface **133a** facing the first accommodating part **131** and a second surface **133b** facing the second accommodating part **132,** and partitions the first accommodating part **131** and the second accommodating part **132.**

According to an embodiment of the present invention, the sealing part **133** may be formed in one body with the first accommodating part **131** and the second accommodating part **132.** According to an embodiment of the present invention, as the adapter **130** is formed by injection molding, the sealing part **133** may be integrally formed with the first accommodating part **131** and the second accommodating part **132.** According to an embodiment of the present invention, the sealing part **133** may be formed of the same material as the first accommodating part **131** and the second accommodating part **132.** The material may be, for example, a plastic material, specifically, polypropylene, polyester, polyethylene (*e.g.,* high density polyethylene (HDPE)), or polyamide (PA66 or nylon 66).

The sample collection device of the present invention may be made of a plastic material, and specifically, may be polypropylene, polyester, polyethylene (for example, high density polyethylene (HDPE)) or polyamide (PA66 or nylon 66).

According to one embodiment, the adapter is made or formed of the same material as the first tube, or is made or formed of a different material from the first tube. When the first tube and the adapter are manufactured from different materials, materials having different properties may be used. For example, the first tube and the adapter may each be manufactured or made using polyethylene and polypropylene, which are materials having different hardness (or stiffness), and specifically, the adapter may be made of polyethylene and the first tube may be made of polypropylene, or the adapter may be made of polypropylene and the first tube may be made of polyethylene. Due to this difference in materials, the sample transport medium may be prevented from leaking along the outer circumferential surface of the first tube while moving to the first tube via the broken sealing part.

The adapter **130** may be formed in, for example, a cylindrical shape, and both end parts may be opened, and the opened both end parts may form openings of the first accommodating part **131** and the second accommodating part **132,** respectively. The diameter of the second accommodating part **132** may be greater or smaller than the diameter of the first accommodating part **131.** Alternatively, the diameters of the second accommodating part **132** and the first accommodating part **131** may be the same.

The sealing part **133** may be formed, for example, in a disk shape whose an edge is connected to an inner circumferential surface of the adapter **130.**

According to one embodiment of the present invention, a thickness of a central portion of the sealing part **133** is thicker than a thickness of an edge portion. The edge portion is a portion connected to the inner circumferential surface of the adapter **130** of the sealing part **133,** and the central portion is a center portion of the sealing part **133.** For example, in the sealing part **133,** the thickness of each of the central portion and the edge portion may be constant to a predetermined thickness, but the thickness of the central portion may be thicker than the thickness of the edge portion, and between the central portion and the edge portion includes a portion where the thickness constantly decreases along the direction from the central portion toward the edge portion. According to an embodiment, the sealing part **133** may be formed such that the second surface **133b** facing the second accommodating part **132** is convex. That is, the first surface **133a** may be flat, the second surface **133b** may convexly protrude, and the sealing part **133** may be formed such that the thickness of the central portion is thicker than the thickness of the edge portion.

The breaking line **134** is a portion where the sealing part **133** is broken, the first accommodating part **131** and the second accommodating part **132** are communicated with each other through the sealing part **133** broken along the breaking line **134,** and the sample transport medium accommodated in the second tube **120** may move to the first tube **110.**

The term "breaking line" used herein refers to a line along which a sealing part is broken, *i.e.,* torn, by contact pressure applied to the sealing part, and is distinguished from a line cut with a sharp blade (see FIGS. 3 and 4).

According to an embodiment, the breaking line **134** may be formed by recessing or grooving the first surface **133a** (see FIG. 3(B), FIG. 4(D), (E), (F)).

The breaking line **134** may be formed outside or inside the portion to which the contact pressure is provided to the sealing part **133.** Alternatively, the breaking line **134** may be formed at a position corresponding to a portion at which the contact pressure is provided to the sealing part **133.** In one embodiment, the breaking line **134** is formed outside a portion where the contact pressure is provided to the sealing part **133.** That is, the breaking line **134** is located outside a portion to which the contact pressure is provided from the center of the sealing part **133.** In addition, the breaking line **134** is located more inward than a portion corresponding to the inner diameter of the second tube **120** opened to the second accommodating part **132** such that the sealing part **133** is broken while the entire or a part of the sealing part **133** moves toward the second accommodating part **132** by the contact pressure.

According to an embodiment of the present invention, the breaking line **134** may be formed in a circular shape. According to one embodiment, the breaking line **134** may be formed in an arc shape. When the breaking line **134** is formed in an arc shape, the central angle of the breaking line **134** (*i.e.,* the central angle of the arc-shaped sector) may be selected within a range from greater than 0° to less than 360°, for example, may be 30°, 45°, 60°, 75°, 90°, 120°, 150°, 180°, 210°, 240°, 270°, 300°, or 330°. The diameter of the breaking line **134** formed in the circular or circular arc may be smaller than the inner diameter of the second tube **120.** Here, the diameter of the breaking line formed as an arc represents the length of the chord connecting both end points of the arc.

Referring to (A) and (D) of FIG. 4, according to an embodiment of the present invention, the breaking line **134** may be formed at the edge portion of the sealing part **133.** That is, when the breaking line **134** formed in the edge portion is broken, the connection between the sealing part **133** and the inner circumferential surface of the adapter **130** may be broken, and the first accommodating part **131** and the second accommodating part **132** may be communicated with each other.

Alternatively, referring to (B) and (E) of FIG. 4, according to an embodiment of the present invention, the breaking line **134** may be formed inside the edge portion of the sealing part **133.** That is, when the breaking line **134** formed inside the edge portion is broken, a hole may be formed in the sealing part **133** and the first accommodating part **131** and the second accommodating part **132** may be communicated with each other. Since the hole formed in the sealing part **133** is located inside the edge portion of the sealing part **133,** the sample transport medium is prevented from leaking without moving from the second tube **120** to the first tube **110.** The first tube **110** inserted into the first accommodating part **131** to break the breaking line **134** formed inside the edge portion may provide a contact pressure to the sealing part **133** inside the breaking line **134.**

More specifically, in order to prevent the sample transport medium from flowing out along the outer circumferential surface of the first tube while moving from the second tube **120** to the first tube **110,** a portion in which the edge portion of the sealing part and the breaking line are connected in (B) and (E) of FIG. 4 may be formed to be inclined toward the first accommodating part.

According to an embodiment, the sealing part **133** may be formed such that a thickness of a portion where the breaking line **134** is formed is thinner than a thickness of the other portion. That is, since the portion where the breaking line **134** is formed is formed to have a thin thickness, the user may easily break the sealing part **133** with a low contact pressure. Referring to (A) and (D) of FIG. 4, the breaking line **134** may be formed at an edge portion having a thinner thickness than that of a central portion of the sealing part **133.** Referring to (B) and (E) of FIG. 4, the breaking line **134** may be formed inside the edge portion of the sealing part **133,** and the sealing part **133** may be formed to have a thin thickness from the edge portion to the portion where the breaking line **134** is formed, and may be formed to be thicker inside the breaking line **134** than the portion where the breaking line **134** is formed. Referring to (C) and (F) of FIG. 4, the breaking line **134** may be formed inside the edge portion of the sealing part **133,** and in the sealing part **133,** a portion between the edge portion and the breaking line **134** and a portion inside the breaking line **134** may be thicker than a portion where the breaking line **134** is formed. According to an embodiment, when the breaking line **134** is formed inside the edge portion of the sealing part **133,** the thickness of the portion where the breaking line **134** is formed may be equal to or different from the thickness of the edge portion between the sealing part **133** and the inner circumferential surface of the adapter **130,** and more specifically, the thickness of the portion where the breaking line **134** is formed may be thinner than the thickness of the edge portion.

As described above, when the sealing part **133** is broken, the first accommodating part **131** and the second accommodating part **132** are communicated with each other, and the sample transport medium accommodated in the second accommodating part **132** is moved to the first accommodating part **131** to be mixed with the collected sample. Hereinafter, a structure in which a contact pressure for breaking the sealing part **133** is provided to the sealing part **133** as the first tube **110** is inserted into the first accommodating part **131** will be described with reference to FIGS. 5 to 10.

Referring to FIGS. 5 and 6, according to an embodiment of the present invention, the sealing part **133** may include a first protrusion part **141** that is protruded from the first surface **133a** and is contacted with the first tube **110** (or supported by (or on) the first tube **110** or supporting the first tube **110**) inserted into the first accommodating part **131** such that the contact pressure provided by the first tube **110** inserted into the first accommodating part **131** breaks the sealing part **133.** That is, the first protrusion part **141** is formed on the first surface **133a** facing the first accommodating part **131** of the sealing part **133,** and the first tube **110** is inserted into the first accommodating part **131** so that the sealing part **133** is supported by the first protrusion part **141,** and thus a contact pressure is provided to the sealing part **133** and the sealing part **133** may be broken.

In this specification, the expression "contact or is contacted with the first tube **110"** used while referring to the first protrusion part **141** may be used interchangeably with "supported by (or on) the first tube **110"** or "supporting the first tube **110".**

According to an embodiment of the present invention, the first protrusion part **141** is located or positioned on the breaking line **134** formed on the first surface **133a,** or is located or positioned inside or outside the breaking line **134** formed on the first surface **133a.** That is, the contact pressure for breaking the sealing part **134** may be provided to the breaking line **134** or may be provided to a region inside or outside of the breaking line **134.**

As described above, the first protrusion part **141** may be formed inside the breaking line **134** such that the contact pressure for breaking the sealing part **133** is provided inside the breaking line **134.** Accordingly, the contact pressure that the first tube **110** provides while being supported by the first protrusion part **141** is provided to a region inside of the breaking line **134.** According to an embodiment, the breaking line **134** may be formed at the edge portion of the sealing part **133,** and the first protrusion part **141** may be formed inside the edge portion. When the first tube **110** is screw-coupled to the first accommodating part **131,** the first tube **110** is supported by the first protrusion part **141** (or supports the first protrusion part **141**) as it moves forward. Therefore, the contact pressure that the first tube **110** provides to the sealing part **133** is preferentially intensively provided to the portion where the first protrusion part **141** of the sealing part **133** is formed as the first tube **110** is supported by the first protrusion part **141** (or supports the first protrusion part **141**), thereby making it easier for the user to break the sealing part **133.**

According to an embodiment of the present invention, the sealing part **133** may include one first protrusion part **141.** Alternatively, according to an embodiment, the sealing part **133** may comprise a plurality of the first protrusion part **141.** When the sealing part **133** includes one first protrusion part **141,** the one first protrusion part **141** may be formed to have the same height so as to be simultaneously supported by the first tube **110,** or a part of one first protrusion part **141** may be formed to have different heights so as to be supported earlier than the others. When the sealing part **133** includes the plurality of the first protrusion part **141,** the plurality of the first protrusion part **141** may be formed to be simultaneously supported by the first tube **110,** or some of the plurality of the first protrusion part **141** may be formed to be supported prior to the others. For example, the plurality of the first protrusion part **141** may be formed to be arranged in the circumferential direction, have different heights, and be sequentially supported by the first tube **110.**

According to an embodiment of the present invention, the first protrusion part **141** may be located or positioned on the first surface **133a** to be supported by an edge portion **110b** of the opened end part **110a** of the first tube **110.** That is, the first protrusion part **141** may be located at the edge portion of the first surface **133a,** and may overlap the edge portion **110b** of the end part **110a** opened in a direction in which the first tube **110** is inserted into the first accommodating part **131.** Accordingly, the edge portion **110b** of the opened end part **110a** of the first tube **110** is supported by the first protrusion part **141,** and a contact pressure is provided to the sealing part **133.**

According to an embodiment, the first protrusion part **141** may be located on a part or a whole of an edge portion of the first surface **133a.** Referring to FIG. 5(A), the first protrusion part **141** may be located on a part of the edge portion of the first surface **133a,** and the first protrusion part **141** may not be located on the others. The first protrusion part **141** positioned at a part of the edge portion of the first surface **133a** may be formed in an arc shape. The central angle of the first protrusion part **141** having the circular arc shape (*i.e*., the central angle of the arc-shaped sector) may be selected within a range from greater than 0° to less than 360°, for example, may be 30°, 45°, 60°, 75°, 90°, 120°, 150°, 180°, 210°, 240°, 270°, 300°, or 330°. FIG. 5A illustrates an embodiment in which the first protrusion part **141** is formed in a circular arc shape having a central angle of about 120 degrees. The first protrusion part **141** located on a whole edge portion of the first surface **133a** may be formed in a circular shape.

According to an embodiment of the present invention, the first protrusion part **141** may have a constant height protruding from the first surface **133a.** Accordingly, the first tube **110** inserted into the first accommodating part **131** may be in surface contact with the first protrusion part **141** having the constant height. When the sealing part **133** includes the plurality of the first protrusion part **141,** all of the plurality of the first protrusion part **141** may have a constant height, and the first tube **110** inserted into the first accommodating part **131** is contacted with all of the plurality of the first protrusion part **141** at the same time. Alternatively, according to an embodiment, the height of the first protrusion part **141** protruding from the first surface **133a** is not constant. Referring to FIG. 5B, the height of the first protrusion part **141** protruding from the first surface **133a** may increase or decrease along the edge portion of the first surface **133a.** Accordingly, the first tube **110** inserted into the first accommodating part **131** is contacted with a portion at which the height of the first protrusion part **141** is highest. When the sealing part **133** includes a plurality of the first protrusion part **141,** each of the first protrusion part **141** may have a height that is not uniform.

FIG. 6 illustrates a process in which the sealing part **133** is broken by the first tube **110** inserted into the first accommodating part **131.** FIG. 6A illustrates a state before the first tube **110** is supported by the first protrusion part **141** (or before the first tube **110** supports the first protrusion part **141**), and FIG. 6B illustrates a state in which the first tube **110,** in which the end part **110a** is completely inserted into the first accommodating part **131,** is supported by the first protrusion part **141** (or the first tube **110** supports the first protrusion part **141**), and a contact pressure is provided so that the sealing part **133** is broken to allow the first accommodating part **131** and the second accommodating part **132** to be communicated with each other. In a state in which the end part **110a** of the first tube **110** is completely inserted into the first accommodating part **131,** only a part of the breaking line **134** may be broken and the others may not be broken. As illustrated in the drawing, the breaking line **134** may be broken only near a portion of the first protrusion part **141** supported by the first tube **110,** and the breaking line **134** may not be broken on the opposite side.

According to an embodiment of the present invention, the first tube **110** may include a second protrusion part **142** formed to protrude in a direction to be inserted into the first accommodating part **131** to be contacted with the sealing part **133** (or to be supported by (or on) the sealing part **133** or to support the sealing part **133**) such that a contact pressure provided by the first tube **110** inserted into the first accommodating part **131** breaks the sealing part **133.**

In this specification, the expression "to be contacted with the sealing part **133"** used while referring to the second protrusion part **142** may be used interchangeably with "to be supported on the sealing part **133"** or "to support the sealing part **133".**

According to an embodiment, the second protrusion part **142** may be located or positioned on an edge portion **110b** of the opened end part **110a** of the first tube **110** (see FIG. 7). Alternatively, according to an embodiment of the present invention, the second protrusion part **142** may be located or positioned on an opening of the first tube **110** (see FIG. 9). Accordingly, as the first tube **110** is inserted into the first accommodating part **131,** the second protrusion part **142** is supported on the first surface **133a,** a contact pressure is provided to the sealing part **133,** and the sealing part **133** is broken.

Depending on the positions of the second protrusion part **142** and the breaking line **134,** the contact pressure for breaking the sealing part **133** may be provided to the breaking line **134** or may be provided to a region inside or outside the breaking line **134.**

According to an embodiment of the present invention, the contact pressure by the second protrusion part **142** may be provided to the breaking line **134** or may be provided to a region inside or outside of the breaking line **134.**

As described above, the breaking line **134** may be formed outside a portion where the second protrusion part **142** is supported by (or on) the first surface **133a** such that the contact pressure for breaking the sealing part **133** is provided to a region inside of the breaking line **134.** According to an embodiment, when the second protrusion part **142** is positioned at the edge portion **110b** of the opened end part **110a** of the first tube **110,** the breaking line **134** may be positioned at the edge portion of the sealing part **133** (see FIG. 8). Alternatively, when the second protrusion part **142** is located on the opening of the first tube **110** according to an embodiment of the present invention, the breaking line **134** may be located inside the edge portion of the sealing part **133** (see FIG. 9).

According to an embodiment, the first tube **110** may include one second protrusion part **142** (see FIGS. 7 and 9). Alternatively, according to one embodiment of the present invention, the first tube **110** may include a plurality of the second protrusion part **142.** When the first tube **110** includes the plurality of the second protrusion part **142,** the plurality of the second protrusion part **142** may be formed to be simultaneously supported on the first surface **133a,** or some of the plurality of the second protrusion part may be formed to be supported earlier than the others. For example, the plurality of the second protrusion part **142** may be disposed in the circumferential direction, have different heights, and be sequentially supported on the first surface **133a.**

According to an embodiment of the present invention, the second protrusion part **142** may have a constant height in a direction in which it is inserted into the first accommodating part **131.** Accordingly, the first surface **133a** and the second protrusion part **142** may be in surface contact with each other. Specifically, the second protrusion part may have a quadrangular or trapezoidal shape. When the first tube **110** includes the plurality of the second protrusion part **142,** all of the plurality of the second protrusion part **142** may have a constant height, and the first surface **133a** is contacted with all of the plurality of the second protrusion part **142** at the same time. Alternatively, according to an embodiment, the second protrusion part **142** may not have a constant height in a direction in which it is inserted into the first accommodating part **131.** Referring to FIGS. 7 and 9, the height at which the second protrusion part **142** protrudes may increase or decrease along the circumferential direction, and the second protrusion part **142** may be formed in a wedge shape with one circumferential side being gentle and the other side being steep. Alternatively, the second protrusion part may have a triangular shape or a pointed shape in which the protruding height increases and then decreases along the circumferential direction. Accordingly, the first surface **133a** is contacted with a portion at which the second protrusion part **142** has the highest height. When the first tube **110** includes a plurality of the second protrusion part **142,** each of the second protrusion part **142** may be formed to have a non-uniform height.

Referring to FIG. 7, the second protrusion part **142** may be located or positioned on the edge portion **110b** of the opened end part **110a** of the first tube **110.** Accordingly, as the first tube **110** is inserted into the first accommodating part **131,** the second protrusion part **142** may be supported on the first surface **133a,** a contact pressure may be provided to the sealing part **133,** and the sealing part **133** may be broken. In this case, according to an embodiment of the present invention, the breaking line **134** is formed at the edge portion of the sealing part **133** (that is, the portion of the sealing part **133** corresponding to the second protrusion part **142** located at the edge portion **110b** of the opened end part **110a** of the first tube **110**) or is formed inside the edge portion of the sealing part **133.** FIG. 8 illustrates a process in which the sealing part **133** is broken by the first tube **110** inserted into the first accommodating part **131.** FIG. 8A illustrates a state before the second protrusion part **142** is supported on the first surface **133a,** and FIG. 8B illustrates a state in which the second protrusion part **142** of the first tube **110,** in which the end part **110a** is completely inserted into the first accommodating part **131,** is supported on the first surface **133a** and provides a contact pressure so that the sealing part **133** is broken, thereby allowing the first accommodating part **131** to communicate with the second accommodating part **132.**

According to an embodiment of the present invention, the second protrusion part **142** is located or positioned on a part of the edge portion **110b** of the opened end part **110a** of the first tube **110.** More specifically, the second protrusion part **142** positioned on a part of the edge portion is formed in an arc shape. More specifically, the second protrusion part **142** formed in the circular arc has a constant protruding height.

Referring to FIG. 9, the second protrusion part **142** may be positioned on the opening of the first tube **110,** and the first tube **110** may include a supporting part **143** for supporting the second protrusion part **142** positioned on the opening. Since the second protrusion part **142** is located on the opening that is more inward than the edge portion **110b** of the first tube **110,** a contact pressure is applied to the sealing part **133** at a position closer to the center (*i.e.,* a position inward than the edge portion of the sealing part **133**). The supporting part **143** supports the second protrusion part **142** while maintaining the opening of the first tube **110** in open state. The supporting part **143** is not limited to the shape illustrated in the drawings, as long as it is connected to the opened end part **110a** of the first tube **110** and has a shape capable of supporting the second protrusion part **142.** As shown in FIG. 9A, the supporting part **143** may form to extend from the inner circumferential surface of the opened end part **110a** of the first tube **110** toward the center, and the second protrusion part **142** may form to protrude from the supporting part **143.** Alternatively, as illustrated in FIG. 9B, the supporting part **143** may include a center part apart from the inner circumferential surface of the opened end part **110a** of the first tube **110** and a connection part connecting the inner circumferential surface of the opened end part **110a** with the center part, and the second protrusion part **142** may form to protrude from the center part. The drawing illustrates an embodiment in which the center part is formed in a circular shape and four connection parts are provided. According to an embodiment of the present invention, the breaking line **134** may be formed at the edge portion of the sealing part **133** or at the inside of the edge portion of the sealing part **133** (*i.e.,* the portion of the sealing part **133** corresponding to the second protrusion part **142** located on the opening of the first tube **110**) such that the contact pressure may be provided to the sealing part **133** by the second protrusion part **142** located on the opening of the first tube **110.**

According to an embodiment, the supporting part **143** may not protrude from the edge portion **110b** of the first tube **110,** and the second protrusion part **142** may form to protrude from the supporting part **143.** FIG. 10 illustrates a process in which the sealing part **133** is broken by the first tube **110** inserted into the first accommodating part **131** based on the embodiment illustrated in FIG. 9A. FIG. 10(A) illustrates a state before the second protrusion part **142** is supported on the first surface **133a,** and FIG. 10(B) illustrates a state in which the second protrusion part **142** of the first tube **110,** in which the end part **110a** is completely inserted into the first accommodating part **131,** is supported on the first surface **133a** and provides a contact pressure so that the sealing part **133** is broken, whereby the first accommodating part **131** and the second accommodating part **132** are communicated with each other.

As described above, the first protrusion part **141** is formed on the first surface **133a** of the sealing part **133** or the second protrusion part **142** is formed on the first tube **110,** such that the contact pressure for breaking the sealing part **133** is concentrated on a portion of the sealing part, thereby allowing the user to break the sealing part **133** more easily.

In an aspect of the present invention, there is provided a method for collecting a sample (the method for collecting a sample of the first aspect), the method comprising: (a) accommodating a collected sample in a first tube; wherein the first tube comprises an opened end part; (b) inserting the first tube accommodating the sample into an adapter coupled with a second tube accommodating a sample transport medium; wherein the second tube comprises an opened end part, wherein the adapter comprises a first accommodating part formed such that the opened end part of the first tube is inserted, a second accommodating part formed to be coupled to the opened end part of the second tube and a sealing part that comprises a first surface facing the first accommodating part and a second surface facing the second accommodating part, and partitions the first accommodating part and the second accommodating part, and wherein a breaking line is formed on the sealing part such that the sealing part is broken by a contact pressure; and (c) breaking the sealing part and moving the sample transport medium accommodated in the second tube to the first tube.

Since the method for collecting a sample of the first aspect is performed by using the sample collection device of the first aspect described above, the common descriptions between them are omitted in order to avoid undue redundancy leading to the complexity of the present specification.

In step of accommodating a collected sample in a first tube, the sample may be accommodated in the first tube using a funnel or a swab device for collecting a sample. For example, in the case of a saliva sample, the collected sample may be accommodated in the first tube by spitting saliva at the opened end part of the first tube or by coupling the funnel to the opened end part of the first tube and then spitting saliva at the funnel. Alternatively, when using the swab device for collecting a sample, the swab device may be located in a sublingual part of the oral cavity to absorb saliva in the fiber layer of the swab device, and then the swab device may be located in the first tube to accommodate the collected sample in the first tube.

The step of coupling the first tube to a adapter coupled to a second tube is a step before the sample transport medium is supplied to the first tube, and the first tube and the adapter formed to be screw coupled to each other are used, so that a user can easily couple the first tube to the adapter by inserting the first tube into a first accommodating part of the adapter and rotating the first tube.

The step of moving the sample transport medium to the first tube is a step of providing the sample transport medium accommodated in the second tube to the sample accommodated in the first tube so as to mix the sample and the sample transport medium, the contact pressure for breaking the sealing part is provided when the user inserts the first tube into the first accommodating part. When the sealing part is broken, the first accommodating part and the second accommodating part of the adapter are communicated with each other, and the sample transport medium accommodated in the second tube is moved to the first tube.

The user may move the sample transport medium to the first tube to provide the sample, and then transport the first tube accommodating the sample and the sample transport medium to a testing site. The user may transport the first tube **110,** which is in a state of being coupled to the adapter **130** to which the second tube **120** is coupled, to the testing site. Alternatively, the user may separate the first tube from the adapter to which the second tube is coupled and transport the sealed first tube to the testing site by, for example, covering the opened end part of the first tube using a tube cap.

According to one embodiment of the present invention, the device is a self-sample collection device. Sample (or specimen) collection using the sample collection device may be easily performed by the general public without the involvement of experts. This is due to convenience and safety of the sample collection device.

Referring to FIGS. 12 and 13, in another aspect of the present invention, there is provided a sample collection device **200** (a sample collection device of a second aspect), comprising: (a) a first tube **110** accommodating a sample; wherein the first tube **110** comprises an opened end part **110a**; and (b) a second tube **120** accommodating a sample transport medium; wherein the second tube **120** comprises an accommodating part **121** formed at one end of the second tube **120** such that the opened end part **110a** of the first tube **110** is inserted, and a sealing part **133** that comprises a first surface **133a** facing the accommodating part **121** and a second surface **133b** facing the other end of the second tube **120** and partitions the second tube **120,** and wherein a breaking line **134** is formed on the sealing part **133** such that the sealing part **133** is broken by a contact pressure.

Since the sample collection device **200** of the second aspect is characterized in that an adapter connecting the first tube and second tube is integrally formed at one end of the second tube, the same reference numerals are used for the same components as the sample collection device **100** of the first aspect described above, a detailed description of common matters will be omitted, and differences will be mainly described.

The inside of the second tube **120** is partitioned by the sealing part **133,** such that one end part forms an accommodating part **121** into which the opened end part **110a** of the first tube **110** is inserted, and the other end part forms an accommodation space **122** accommodating the sample transport medium.

Meanwhile, referring to FIG. 14, according to one embodiment of the present invention, the other end of the second tube **120** is an opened end part. According to an embodiment of the present invention, the sample collection device **200** further includes a tube cap **230** coupled to the opened end part of the second tube **120.** The sample transport medium may be filled through the opened end part of the second tube **120** and may be accommodated in the second tube **120** by coupling the tube cap **230.** That is, after the sample transport medium is supplied to the accommodating space **122** through the opened end part of the second tube **120,** the tube cap **230** may be coupled to the opened end part to seal the accommodating space **122.** The tube cap **230** may be screwed to the opened end part.

Then, as the opened end part **110a** of the first tube **110** is inserted into the accommodating part **121,** a contact pressure for breaking the sealing part **133** is provided to the sealing part **133.** When the sealing part **133** is broken, the sample transport medium accommodated in the second tube **120** is moved into the first tube **110** through the broken sealing part **133.**

FIGS. 13(A) and (B) illustrate an embodiment in which the first protrusion part **141** is formed on the first surface **133a** of the sealing part **133,** but the present disclosure is not limited thereto. For example, a second protrusion part may be formed on the edge portion **110b** of the opened end part **110a** of the first tube **110,** or a second protrusion part on the opening of the first tube **110** and a supporting part for supporting the second protrusion part may be formed.

According to an embodiment of the present invention, the sample is selected from the group consisting of a saliva sample, a saliva swab sample, a nasopharyngeal swab sample, an oropharyngeal swab sample, a nasal sample, a nasal swab sample, a nostril sample and a nostril swab sample.

According to one embodiment, the device further comprises a funnel to be coupled to the opened end part of the first tube.

According to one embodiment of the present invention, the device further comprises a tube cap covering the opened end part of the first tube.

According to an embodiment, the device further comprises a swab device for collecting a sample.

According to one embodiment of the present invention, the first tube and the accommodating part are coupled together by their screw threads.

According to an embodiment, the sample transport medium moves from the second tube to the first tube after the sealing part is broken.

According to one embodiment of the present invention, the contact pressure is provided by the first tube inserted into the accommodating part.

According to one embodiment, the contact pressure is provided as the first tube is inserted into the accommodating part and moves forward.

According to one embodiment of the present invention, the sealing part is formed in one body with the second tube.

According to one embodiment, the sealing part is formed or made of the same material as the second tube.

According to one embodiment of the present invention, the second tube is formed or made of the same material as the first tube or is formed or made of a different material from the first tube. When the first tube and the second tube are manufactured using different materials, materials having different properties may be used. For example, the first tube and the second tube may be made of polyethylene and polypropylene having different properties, respectively and specifically, the second tube may be made of polyethylene, the first tube may be made of polypropylene, or the second tube may be made of polypropylene, and the first tube may be made of polyethylene. Due to this difference in materials, the sample transport medium may be prevented from leaking along the outer circumferential surface of the first tube while moving to the first tube via the broken sealing part.

According to one embodiment of the present invention, a thickness of a central portion of the sealing part is thicker than a thickness of an edge portion.

According to one embodiment, the sealing part is formed such that the second surface is convex.

According to an embodiment of the present invention, the breaking line is formed on an edge portion of the sealing part.

According to an embodiment, the breaking line is formed inside an edge portion of the sealing part.

According to one embodiment of the present invention, the sealing part is formed such that a thickness of a portion where the breaking line is formed is thinner than a thickness of the other portion.

According to an embodiment, the sealing part comprises a first protrusion part that is protruded from the first surface and is contacted with the first tube (or is supported by (or on) the first tube or supports the first tube) inserted into the accommodating part such that the contact pressure provided by the first tube inserted into the accommodating part breaks the sealing part.

According to one embodiment of the present invention, the sealing part comprises a plurality of the first protrusion parts.

According to one embodiment, the first protrusion part is located on a breaking line formed on the first surface or is located inside or outside of the breaking line formed on the first surface.

According to an embodiment of the present invention, the first protrusion part is located on the first surface to be supported on an edge portion of the opened end part of the first tube.

According to one embodiment, the first protrusion part is located on the edge portion of the first surface.

According to an embodiment of the present invention, the first protrusion part is located on a part or a whole of an edge portion of the first surface.

According to one embodiment, the first protrusion part has a constant height protruding from the first surface.

According to one embodiment of the present invention, the height of the first protrusion part protruding from the first surface is not constant.

According to an embodiment, the first tube comprises a second protrusion part formed to protrude in a direction to be inserted into the accommodating part to be contacted with the sealing part (or to be supported by (or on) the sealing part or to support the sealing part) such that a contact pressure provided by the first tube inserted into the accommodating part breaks the sealing part.

According to one embodiment of the present invention, the first tube comprises a plurality of second protrusion parts.

According to one embodiment, the second protrusion part is located on an edge portion of the opened end part of the first tube.

According to one embodiment of the present invention, the second protrusion part is located on an opening of the first tube, and the first tube comprises a supporting part for supporting the second protrusion part.

According to one embodiment, the second protrusion part has a constant height protruding in a direction to be inserted into the accommodating part.

According to an embodiment of the present invention, the height of the second protrusion part protruding in a direction to be inserted into the accommodating part is not constant.

According to one embodiment, the device is a self-sample collection device.

In another aspect of the present invention, there is provided a method for collecting a sample comprising (the method for collecting a sample of the second aspect): (a) accommodating a collected sample in a first tube; wherein the first tube comprises an opened end part; and (b) inserting the first tube accommodating the sample into a second tube accommodating a sample transport medium; wherein the second tube comprises an accommodating part formed at one end of the second tube such that the opened end part of the first tube is inserted and a sealing part that comprises a first surface facing the accommodating part and a second surface facing the other end of the second tube and partitions the second tube, and wherein a breaking line is formed on the sealing part such that the sealing part is broken by a contact pressure, and (c) breaking the sealing part and moving the sample transport medium accommodated in the second tube to the first tube.

Since the method for collecting a sample of the second aspect is performed by using the sample collection device of the second aspect described above, the common descriptions between them are omitted in order to avoid undue redundancy leading to the complexity of the present specification.

Further, the common contents between the methods for collecting a sample of the first and second aspects are omitted in order to avoid undue redundancy leading to the complexity of the present specification.

A funnel or a swab device for collecting a sample is used to accommodate a sample in the first tube, the first tube is inserted into the accommodating part of the second tube which accommodates a sample transport medium, a contact pressure is provided by the inserted first tube, and the sealing part is broken. The sample transport medium may be moved from the second tube to the first tube through the broken sealing part so as to mix the sample and the sample transport medium and then transfer the sample collection device to an inspection site or the first tube and the second tube may be separated and then the opened end part of the first tube may be sealed with the tube cap so as to transfer the sealed first tube to the inspection site.

The description of the present invention in the specification describes preferred embodiments, and the present invention is not limited to such embodiments. Those skilled in the art may make various changes and modifications to the above embodiments without departing from the technical spirit of the present invention, and the technical spirit of the present invention includes all of the various changes and modifications.
**100:** Sample collection device **110:** First tube
**110a:** Opened end part **110b:** Edge portion
**120:** Second tube **120a:** Opened end part
**121:** Accommodating part **122:** Space accommodating sample transport medium
**130:** Adapter **131:** First accommodating part
**132:** Second accommodating part **133:** Sealing part
**133a:** First surface **133b:** Second surface
**134:** Breaking line **141:** First protrusion part
**142:** Second protrusion part **143:** supporting part
**210:** Funnel **220:** Swab device for collecting a sample
**230:** Tube cap **301:** Protrusion
**302:** Groove

## Claims

1. A sample collection device, comprising:
(a) a first tube accommodating a sample; wherein the first tube comprises an opened end part;
(b) a second tube accommodating a sample transport medium; wherein the second tube comprises an opened end part; and
(c) an adapter; wherein the adapter comprises a first accommodating part formed such that the opened end part of the first tube is inserted, a second accommodating part formed to be coupled to the opened end part of the second tube and a sealing part that comprises a first surface facing the first accommodating part and a second surface facing the second accommodating part, and partitions the first accommodating part and the second accommodating part, and wherein a breaking line is formed on the sealing part such that the sealing part is broken by a contact pressure.

2. The sample collection device according to claim 1, wherein the sample is selected from the group consisting of a saliva sample, a saliva swab sample, a nasopharyngeal swab sample, an oropharyngeal swab sample, a nasal sample, a nasal swab sample, a nostril sample and a nostril swab sample.

3. The sample collection device according to claim 1, wherein the device further comprises a funnel to be coupled to the opened end part of the first tube.

4. The sample collection device according to claim 1, wherein the device further comprises a tube cap covering the opened end part of the first tube.

5. The sample collection device according to claim 1, wherein the device further comprises a swab device for collecting a sample.

6. The sample collection device according to claim 1, wherein the first tube and the first accommodating part are coupled together by their screw threads.

7. The sample collection device according to claim 1, wherein the sample transport medium is accommodated in a space formed by coupling the opened end part of the second tube and the second accommodating part partitioned by the sealing part.

8. The sample collection device according to claim 1, wherein the sample transport medium moves from the second tube to the first tube after the sealing part is broken.

9. The sample collection device according to claim 1, wherein the contact pressure is provided by the first tube inserted into the first accommodating part.

10. The sample collection device according to claim 9, wherein the contact pressure is provided as the first tube is inserted into the first accommodating part and moves forward.

11. The sample collection device according to claim 1, wherein the sealing part is formed in one body with the first accommodating part and the second accommodating part.

12. The sample collection device according to claim 1, wherein the sealing part is made of the same material as the first accommodating part and the second accommodating part.

13. The sample collection device according to claim 1, wherein the adapter is made of the same material as the first tube or is made of a different material from the first tube.

14. The sample collection device according to claim 1, wherein a thickness of a central portion of the sealing part is thicker than a thickness of an edge portion.

15. The sample collection device according to claim 14, wherein the sealing part is formed such that the second surface is convex.

16. The sample collection device according to claim 1, wherein the breaking line is formed on an edge portion of the sealing part.

17. The sample collection device according to claim 1, wherein the breaking line is formed inside an edge portion of the sealing part.

18. The sample collection device according to claim 1, wherein the sealing part is formed such that a thickness of a portion where the breaking line is formed is thinner than a thickness of the other portion.

19. The sample collection device according to claim 1, wherein the sealing part comprises a first protrusion part that is protruded from the first surface and is contacted with the first tube inserted into the first accommodating part such that the contact pressure provided by the first tube inserted into the first accommodating part breaks the sealing part.

20. The sample collection device according to claim 19, wherein the sealing part comprises a plurality of the first protrusion parts.

21. The sample collection device according to claim 19, wherein the first protrusion part is located on a breaking line formed on the first surface or is located inside or outside of the breaking line formed on the first surface.

22. The sample collection device according to claim 19, wherein the first protrusion part is located on the first surface to be supported on an edge portion of the opened end part of the first tube.

23. The sample collection device according to claim 19, wherein the first protrusion part is located on a part or a whole of an edge portion of the first surface.

24. The sample collection device according to claim 19, wherein the first protrusion part has a constant height protruding from the first surface.

25. The sample collection device according to claim 19, wherein the height of the first protrusion part protruding from the first surface is not constant.

26. The sample collection device according to claim 1, wherein the first tube comprises a second protrusion part formed to protrude in a direction to be inserted into the first accommodating part to be contacted with the sealing part such that a contact pressure provided by the first tube inserted into the first accommodating part breaks the sealing part.

27. The sample collection device according to claim 26, wherein the first tube comprises a plurality of second protrusion parts.

28. The sample collection device according to claim 26, wherein the second protrusion part is located on an edge portion of the opened end part of the first tube.

29. The sample collection device according to claim 26, wherein the second protrusion part is located on an opening of the first tube, and the first tube comprises a supporting part for supporting the second protrusion part.

30. The sample collection device according to claim 26, wherein the second protrusion part has a constant height protruding in a direction to be inserted into the first accommodating part.

31. The sample collection device according to claim 26, wherein the height of the second protrusion part protruding in a direction to be inserted into the first accommodating part is not constant.

32. The sample collection device according to claim 1, wherein the second tube and the second accommodating part are coupled together by their screw threads.

33. The sample collection device according to claim 1, wherein the second tube comprises a plurality of protrusions disposed on an outer circumferential surface, and the second accommodating part comprises a plurality of grooves disposed on an inner circumferential surface and engaged with the protrusions.

34. The sample collection device according to claim 33, wherein the protrusion and the groove are formed to rotate only in a direction in which the second tube is coupled to the second accommodating part.

35. The sample collection device according to claim 1, wherein the device is a self-sample collection device.

36. A sample collection device, comprising:
(a) a first tube accommodating a sample; wherein the first tube comprises an opened end part; and
(b) a second tube accommodating a sample transport medium; wherein the second tube comprises an accommodating part formed at one end of the second tube such that the opened end part of the first tube is inserted and a sealing part that comprises a first surface facing the accommodating part and a second surface facing the other end of the second tube and partitions the second tube, and wherein a breaking line is formed on the sealing part such that the sealing part is broken by a contact pressure.

37. A method for collecting a sample, comprising:
(a) accommodating a collected sample in a first tube; wherein the first tube comprises an opened end part;
(b) inserting the first tube accommodating the sample into an adapter coupled with a second tube accommodating a sample transport medium; wherein the second tube comprises an opened end part, wherein the adapter comprises a first accommodating part formed such that the opened end part of the first tube is inserted, a second accommodating part formed to be coupled to the opened end part of the second tube and a sealing part that comprises a first surface facing the first accommodating part and a second surface facing the second accommodating part, and partitions the first accommodating part and the second accommodating part, and wherein a breaking line is formed on the sealing part such that the sealing part is broken by a contact pressure; and
(c) breaking the sealing part and moving the sample transport medium accommodated in the second tube to the first tube.
